# FASCICULE DE BREVET EUROPEEN

(11) **EP 0 808 308 B1**
(45) Date de publication et mention de la délivrance du brevet: **07.03.2001**
(21) Numéro de dépôt: 96943113.9
(22) Date de dépôt: 04.12.1996
(51) Int. Cl.: C07D 231/12

(54) **PROCEDE DE SEPARATION DE CARBINOLS**
VERFAHREN ZUR ABTRENNUNG VON CARBINOLEN
METHOD FOR SEPARATING CARBINOLS

(30) Priorité: 06.12.1995 FR 9514414
(43) Date de publication de la demande: 26.11.1997
(73) Titulaire: LABORATORIOS DEL DR. ESTEVE, S.A., 08026 Barcelona (ES)
(72) Inventeur: FRIGOLA CONSTANSA, Jordi, E-08013 Barcelone (ES); BERROCAL ROMERO, Juana Maria, E-08940 Cornella De Llobregat (ES); CUBERES ALTISENT, Maria Rosa, E-08190 Saint Cugat del Valles (ES); GOTOR SANTAMARIA, Vicente, E-33008 Oviedo (ES)
(74) Mandataire: Le Forestier, Eric
(86) Numéro de dépôt international: EP9605596
(87) Numéro de publication internationale: WO9720817

(56) Documents cités:
- US-A- 4 963 492
- CHEMICAL ABSTRACTS, vol. 119, no. 7, 16 Août 1993 Columbus, Ohio, US; abstract no. 72535b, HUESO-RODRIGUEZ,J.A. ET AL.: "Preparation of the enantiomers of the analgesic E-3710" XP002011469 cité dans la demande & BIOORG. MED. CHEM. LETT., vol. 3(2), 1993, pages 269-272,
- SHKOLNIK AND GUTMAN: "Resolution of Racemic Sterically Hindered Secondary Alcohols via Enzymatic Alcoholysis of Their Esters. The First Enzymatic Preparation of Optically Pure 2,2,2-Trifluoro-1-(9-Anthryl)Ethanols", BIORGANIC & MEDICINAL CHEMISTRY, , 1994, Vol. 2, no. 7, pages 567 à 572

## Description

La présente invention concerne un procédé pour séparer un carbinol de formule 1 en ses énantiomères, ainsi que la racémisation d'un de ceux-ci en effectuant un procédé séquentiel dans le but d'obtenir l'énantiomère désiré avec des rendements élevés. Les stéréo-isomères de 1 sont les composés clés pour la synthèse des énantiomères du composé de formule 2

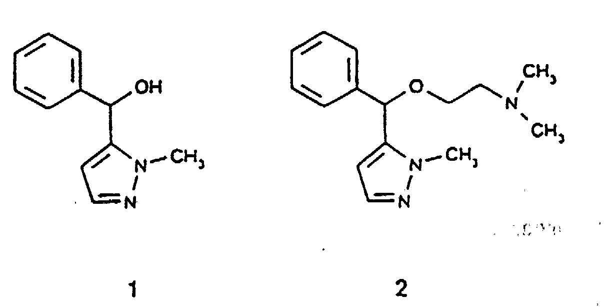

Le (±)-5-{[(N,N-diméthylaminoéthoxy)-phényl]méthyl}-1-méthyl-1H-pyrazole, de formule 2, est un composé avec des propriétés analgésiques, actuellement en phase d'étude clinique décrit dans le brevet européen EP 289 380. Les deux énantiomères de 2 ont été synthétisés et évalués comme analgésiques [J.A.Hueso, J. Berrocal, B. Gutiérrez, A.J. Farré et J. Frigola, Bioorganic & Medicinal Chemistry Letters, 1993, 3, 269-272], et il en est résulté que l'énantiomère dextrogyre est le plus actif.

Les énantiomères (+)-2 et (-)-2 sont obtenus, respectivement, par alkylation de (+)-1 et (-)-1. Le stéréoisomère (+)- 1 a été obtenu avec un très bas rendement à partir de (R)-mandélate d'éthyle, ce qui a déterminé de cette façon la configuration absolue (R)-(+)-1. Les énantiomères de 1 ont été aussi obtenus par des procédés complexes de séparation par chromatographie sur colonne ou de cristallisations fractionnés, des esters diastéréoisoméres formés par réaction de 1 avec l'acide (+)-O-acétylmandélique. Les rendements ont été de 22 % pour l'énantiomère (-)-1 et de 25 % pour l'énantiomère (+)-1.

D'autre part, l'utilisation de biocatalyseurs appliqués à la séparation de mélanges racémiques a été amplement décrite [a)"Microbial reagents in organic synthesis", édité par Stefano Servi, Kluwer Academic Publishers, Londres, 1992. b) "Enzymes in synthetic organic chemistry" édité par C.H. Wong et G.M. Whitesides, Elsevier Science, Oxford 1994. c) "Biotransformations in Organic Chemistry", édité par K. Faber, Lange et Springer, 1995].

Il existe de nombreuses classes distinctes d'enzymes utilisées pour la séparation de stéroisomères, y compris les hydrolases (en particulier les lipases, les protéases et les estérases), les liases et les oxydoréductases. Les hydrolases sont parmi les enzymes les plus attractives pour être utilisées dans les séparations, car elles se trouvent disponibles commercialement à bas coût et certaines d'entre elles montrent une tolérance raisonnable aux solvants organiques.

L'utilisation de solvants organiques dans les réactions catalysées par enzymes présente divers avantages : a) la plus grande partie des substrats organiques sont plus solubles dans les solvants organiques que dans l'eau; b) la récupération des produits de réaction est extraordinairement facilitée ; c) les enzymes sont recueillies avec facilité pour être réutilisées; d) dans certains cas, l'énantiosélectivité est élevée. Malgré les avantages d'utilisation des enzymes dans des solvants organiques, il y a cependant aussi certains désavantages ; a) la recherche d'un solvant adapté ; b) la faible rapidité de réaction ; c) la diminution de la pureté optique du produit désiré dans ces réactions de nature réversible [pour révision voir : a) A.M. Klibanov, Trends Biochem. Sci., 1989, 14, 141. b) C.S. Chen, C.J. Sih, Angew. Chem. Int. Ed. Engl., 1989, 28, 695]. La constatation définitive que certaines enzymes peuvent agir dans des solvants organiques a été une des causes principales de l'augmentation spectaculaire, durant la dernière décénie, de l'emploi des biotransformations pour la préparation de produits d'intérêt thérapeutique et industriel [pour révision voir : a) A.N. Collins, G.N. Sheldrake, S. Crosby, "Chirality in Industry", Wiley, Londres, 1992. b) S.C. Stinson, Chem. & Eng. News, 1994, 38. c) A.L. Margolin, Enzyme Microb. Technol. 1993, 15, 266].

Le brevet US 4,963,492 décrit la résolution enzymatique d'un alcool secondaire racémique de formule 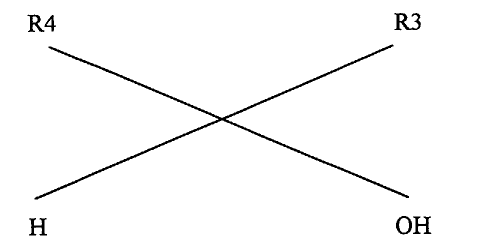 dans lequel R₃ et R₄ forment un cycle, ou dans lequel R₃ représente un radical alkyle ou cycloalkyle et R₄ représente divers substituants qui incluent un noyau aromatique. Cependant, dans ce brevet, il s'agit d'alcools secondaires stériquement peu encombrés en ce sens où R₃ et R₄ ne représentent jamais un noyau aromatique.

L'objet de la présente invention consiste à proposer un procédé commercialement utile pour l'obtention du stéréo-isomère dextrogyre (R)-(+)-1, procédé qui pourrait être également utile pour obtenir le stéro-isomère lévogyre (S)-(-)-1.

Le procédé auquel se réfère la présente invention se base sur la biocatalyse, en utilisant un biocatalyseur pour effectuer de façon sélective une transestérification entre l'alcool racémique 1 et un ester de formule 3, dans laquelle R₁ représente un radical méthyle ou éthyle et R₂ représente un radical vinyle ou isopropényle. Au moyen d'une enzyme qui permet la stéréosélectivité appropriée, on peut obtenir un mélange réactionnel qui contient l'énantiomère de 1 qui n'a pas réagi, en même temps que l'ester de formule 4, dans laquelle R₁ représente un radical méthyle ou éthyle, formé à partir de l'autre énantiomère de 1.

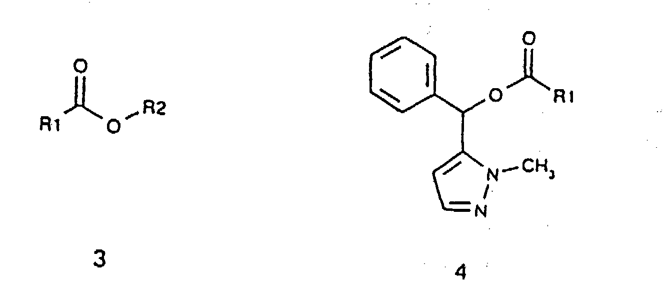

La séparation et la récupération de l'alcool qui n'a pas réagi et de l'ester formé permet donc une utilisation relativement facile des procédés chimiques conventionnels, tels que la chromatographie ou la cristallisation. Un autre aspect de l'invention consiste à effectuer l'hydrolyse de l'ester obtenu en utilisant un catalyseur acide ou basique, de façon qu'il se produise une racémisation, en récupérant ainsi le composé 1, ou la rétention de la configuration. Dans le cas où on obtient le mélange racémique 1, celui-ci peut être traité ensuite avec le biocatalyseur mentionné pour donner lieu à un procédé de transestérification comme décrit précédemment et ainsi successivement.

De cette façon, on obtient une transformation quasi complète du substrat 1 racémique donnant l'énantiomère désiré.

La stratégie utilisée dans la présente invention consiste en la combinaison séquentielle d'une transestérification enzymatique du carbinol 1 et d'une transformation chimique avec racémisation. Les enzymes les plus appropriées pour effectuer la transestérification sont les hydrolases, principalement les lipases produites par micro-organismes, aussi bien libres qu'immobilisées. Les esters utilisés comme agents acylants sont les esters d'énols de formule 3 dans laquelle R₁ représente un radical méthyle ou éthyle et R₂ représente un radical vinyle ou isopropényle. La réaction de transestérification s'effectue en l'absence d'un solvant ou au sein d'un solvant approprié, comme l'hexane, le cyclohexane, le toluène, l'acétone, le dioxanne, le tétrahydrofuranne, l'éthanol, etc., à une température comprise entre 20°C et la température de reflux durant le temps nécessaire pour qu'il se produise la transformation, ce temps pouvant osciller entre 6 heures et 48 heures. L'addition de tamis moléculaires au milieu de réaction peut augmenter l'activité et diminuer la teneur en eau. La progression de la réaction d'acylation se contrôle facilement par résonance magnétique nucléaire de proton. L'énantiomère de 1 qui n'a pas réagi se sépare de l'autre énantiomère estérifié 4 par chromatographie sur colonne de gel de silice ou par cristallisation dans un solvant approprié. La pureté optique est analysée par HPLC (chromatographie liquide de haute résolution) chirale.

L'énantiomère estérifié 4 est hydrolysé pour donner lieu ensuite à la formation du carbinol racémique 1 ou du carbinol correspondant homochiral. L'hydrolyse s'effectue respectivement en milieu acide ou en milieu basique, à une température comprise entre 60°C et la température de reflux pendant un temps compris entre 2 et 24 heures.

Par alkylation avec du diméthylaminochloroéthane, dans des conditions de transfert de phase, des stéréo-isomères (+)-1 et (-)-1, et par traitement ultérieur avec l'acide citrique, on obtient respectivement les stéréo-isomères (+)-2.citrate et (-)-2.citrate.

La description qui précède le procédé de transestéréfication du carbinol racémique 1 pour obtenir un carbinol homochiral et de l'hydrolyse de l'ester homochiral formé pour obtenir l'alcool correspondant homochiral ou bien l'alcool racémique, ainsi que la description détaillée des exemples qui sont indiqués par la suite, donnés uniquement à titre d'illustration, ne doivent limiter d'aucune façon le cadre de la présente invention.

### Exemple 1.

### Séparation de (±)-5-(phényl) hydroxyméthyl-1-méthyl-1H-pyrazole, (±)-1, avec la lypase PS :

Un mélange de 100 g de carbinol ()-1, de 50 g de lipase PS activée, commercialisée par AMANO PHARMACEUTICAL COMPANY Ltd. (NAGOYA - JP), de 50 g de tamis moléculaires de 3 Å activés et de 1000 ml d'acétate de vinyle est agité à 60°C pendant 24 heures. Après vérification par 1H-RMN que le substrat a été acétylé à 55 %, on filtre pour recueillir la Iipase et les tamis moléculaires et on évapore l'acétate de vinyle à pression réduite. On dissout le résidu dans du cyclohexane, d'où on obtient par cristallisation 36 g (72 %) de (+)-5-(phényl)hydrométhyl-1-méthyl-1H-pyrazole, (+)-1, avec une pureté optique supérieure à 96 % (excès énantiomérique déterminé par HPLC=92 %); point de fusion : 80-83°C; IR (KBr) 3237, 1456, 1394, 1294, 1193, 1058, 1006, 785, 765, 708, 701 cm-1 ; [α] _{D}+16,2 (c = 1, CHCl₃). L'évaporation du solvant donne 76,5 g de, majoritairement, (-)-5-(phényl)méthylcarbonyloxyméthyl-1-méthyl-1H-pyrazole, (-)-4, où R₁ représente un radical méthyle.

### Exemple 2.

### Hydrolyse et racémisation de (-) -5-(phényl)méthylcarbonyloxyméthyl-1-méthyl-1H-pyrazole, (-)-4:

76,5 g de (-)-5-(phényl)méthylcarbonyloxyméthyl-1-méthyl-1H-pyrazole, (-)-4 sont mis à reflux au sein de 300 ml d'acide chlorhydrique 6N pendant 12 heures, on filtre en chauffant et on alcalinise la solution avec de l'hydroxyde d'ammonium pour obtenir 61,4 g de carbinol racémique (±)-5-(phényl)hydroxyméthyl-1-méthyl-1H-pyrazole, (±)-1, point de fusion 105-106°C ; IR (KBr) 3225, 1457, 1398, 1208, 1200, 1018, 1009, 794, 751, 702 cm-1 ; excès énantiomérique déterminé par HPLC = 0.

### Exemple 3.

### Séparation de (±)-5-(phényl)hydroxyméthyl-1-méthyl-1H-pyrazole, (+)-1, avec la lipase PS :

Un mélange de 61,4 g de carbinol (+)-1 obtenus dans l'exemple 2, 30,7 g de lipase PS activée, commercialisée par AMANO PHARMACEUTICAL COMPANY Ltd. (NAGOYA - JP), 30,7 g de tamis moléculaires de 3 Å activés et 600 ml d'acétate de vinyle sont agités à 56°C pendant 36 heures. Après vérification avec H-RMN que le substrat a été acétylé à 53 %, on filtre pour recueillir la lipase et les tamis moléculaires et on évapore l'acétate de vinyle à pression réduite. Le résidu est dissous dans le cyclohexane, d'où l'on obtient par cristallisation 21,7 g (71%) de (+)-5-(phényl) hydroxyméthyl-1-méthyl-1H-pyrazole, (+)-1 avec une pureté optique supérieure à 95 %, point de fusion : 79-85°C [α]_{D}+16,0 (c = 1, CHCl₃). L'évaporation du solvant donne 45,8 g de, majoritairement, (-)-5-(phényl)méthylcarbonyloxyméthyl-1-méthyl-1H-pyrazole, (-)-4, où R₁ représente un radical méthyle.

### Exemple 4.

### Séparation de (+)-5-(phényl)hydroxyméthyl-1-méthyl-1H-pyrazole, (±)-1, avec lipase PS :

Un mélange de 3,5 g de carbinol (±)-1, 7,4 g de lipase PS activée, commercialisée par AMANO PHARMACEUTICAL COMPANY Ltd. (NAGOYA-JP) et 100 ml d'acétate de vinyle est agité à 62°C pendant 13 heures. Après vérification par 1H-RMN que le substrat a été acétylé à 50 %, on filtre pour recueillir la lipase et les tamis moléculaires et on évapore l'acétate de vinyle à pression réduite. On chromatographie le résidu sur colonne avec gel de silice en éluant avec éther diéthylique:hexane (2:1) pour obtenir 2,1 g (98%) de (-)-5- (phényl) méthylcarbonyloxyméthyl-1-méthyl-1H-pyrazole, (-)-4, où R₁ représente un radical méthyle. En éluant ensuite avec de l'éther diéthylique, on obtient 1,7 g (98 %) de (+)-5-(phényl)hydroxyméthyl-1-méthyl-1H-pyrazole, (+)-1, avec un excès énantiomérique déterminé par HPLC = 94 %.

### Exemple 5.

### Hydrolyse avec rétention de configuration de (-)-5-(phényl) méthylcarbonyloxyméthyl-1-méthyl-1H-pyrazole, (-)-4:

2,3 g de (-)-5-(phényl)méthylcarbonyloxyméthyl-1-méthyl-1H-pyrazole, (-)-4, sont mis à reflux au sein de 4 ml d'hydroxyde de sodium à 20 % et 10 ml d'éthanol durant 2 heures, on évapore l'éthanol, on ajoute 10 ml d'eau, on extrait avec de l'éther diéthylique, on sèche avec du sulfate de magnésium, on filtre et à partir de la solution, on obtient 1,8 g (95 %) g de (-)-5-(phényl)hydroxyméthyl-1-méthyl-1H-pyrazole, (-)-1, avec une pureté optique supérieure à 94 %, [α] _{D} -16,1 (c = 1, CHCl₃).

### Exemple 6.

Obtention de citrate de (+)-5- {[(N,N-dimétylaminoéthoxy)-phényl] méthyl}-1-méthyl-1H-pyrazole, (+)-2. citrate.

On met à reflux pendant 7 heures un mélange de 12,7 g de (+)-5-(phényl)hydroxyméthyl-1-méthyl-1H-pyrazole, (+)-1, dans 250 ml de toluène, 125 ml d'hydroxyde de sodium à 50 %, 3 g de chlorure de triétylbutylamonium et 14,6 g de chlorhydrate de diméthylaminochloroéthane. Du mélange froid on extrait avec du toluène 16,2 g (92,6 %) de (+)-5- {[(N,N-diméthylaminoéthoxy)-phényl] méthyl} -1-méthyl-1H-pyrazole, (+)-2.

On agite à 40°C un mélange de 15 g de (+)-5-{[(N,N-diméthylaminoéthoxy)-phényl]méthyl}-1-méthyl-1H- pyrazole, (+)-2, et 13,5 g d'acide citrique monohydrate dans l'éthanol jusqu'à dissolution totale. De cette solution on récupère par cristallisation 24,7 g (94,5 %) de citrate de (+)-5- {[(N,N-diméthylaminoéthoxy)-phényl]méthyl} -1-méthyl-1H-pyrazole, (+)-2. citrate, point de fusion 129-131°C, [α]_{D} + 8,3 (c = 1, H₂O).

### Exemple 7.

Obtention de citrate de (-)-5-{[(N,N-diméthylaminoéthoxy) phényl] méthyl} -1-méthyl-1H-pyrazole, (-)-2. citrate.

En procédant de façon analogue à l'exemple 6, et en utilisant l'énantiomère (-)-5-(phényl)hydroxyméthyl-1-méthyl-1H-pyrazole, (-)-1, comme produit de séparation, on obtient le (-)-5-{[(N,N-diméthylaminoéthoxy)-phényl]méthyl} -1-méthyl-1H-pyrazole, (-)-2 citrate, point de fusion 128-130°C [α] _{D} -8,2 (c = 1, H₂O).

## Revendications

1. Procédé pour préparer de façon prédominante l'énantiomère (R)-(+)-5-(phényl)hydroxyméthyl-1-méthyl-1H-pyrazole, (R)-(+)-1, par séparation du mélange racémique (±)-5-(phényl)hydroxyméthyl-1-méthyl-1H-pyrazole, de formule 1, qui se caractérise par un procédé séquentiel qui comprend l'utilisation d'une lipase provenant d'un microorganisme en une réaction de transestérification entre le mélange racémique de formule 1 et un ester de formule 3 dans lequel R₁ représente un radical méthyle ou éthyle et R₂ représente un radical vinyle ou isopropényle, ainsi que l'hydrolyse de l'ester formé, (S)-(-)-5-(phényl)alkylcarbonyloxyméthyl-1-méthyl-1H-pyrazole, de formule (S)-(-)-4 dans laquelle R₁ représente un radical méthyle ou éthyle.

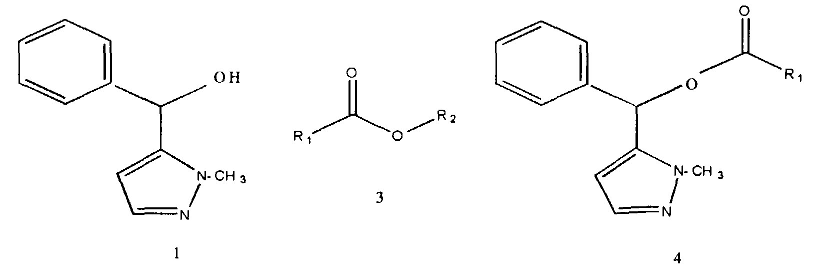

2. Procédé selon la revendication 1, qui se caractérise par l'utilisation d'une lipase provenant d'un microorganisme pour agir comme catalyseur dans une réaction de transestérification entre un ester de formule 3, dans laquelle R₁ représente un radical méthyle ou éthyle et R₂ représente un radical vinyle ou isopropényle, et l'énantiomère (S)-(-)-5-(phényl)hydroxyméthyl-1-méthyl-1H-pyrazole, (S)-(-)-1, ce qui donne lieu à la formation de (S)-(-)-5-(phényl)alkylcarbonyloxyméthyl-1-méthyl-1H-pyrazole de formule (S)-(-)-4 dans laquelle R₁ représente un radical méthyle ou éthyle, et en récupérant l'énantiomère (R)-(-)-5-(phényl)hydroxyméthyl-1-méthyl-1H-pyrazole, (R)-(+)-1, sans réagir.

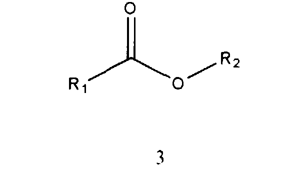

3. Procédé selon l'une des revendications 1 et 2 qui se caractérise par l'utilisation de l'acétate de vinyle, de formule 3, dans laquelle R₁ représente un radical méthyle et R₂ représente un radical vinyle, comme réactif et comme solvant dans la réaction de transestérification.

4. Procédé selon l'une des revendications 1, 2 et 3 qui se caractérise par l'utilisation d'un solvant peu polaire, comme par exemple le cyclohexane, pour cristalliser l'énantiomère (R)-(+)-5-(phényl) hydroxyméthyl-1-méthyl-1H-pyrazole, (R)-(+)-1, de la solution qui contient en outre l'ester (S)-(-)-5-(phényl)méthylcarbonyloxyméthyl-1-méthyl-1H-pyrazole, de formule (S)-(-)-4 dans laquelle R₁ représente un radical méthyle.

5. Procédé selon l'une des revendications 1, 2 et 3, qui se caractérise par le fait que l'on effectue une chromatographie en colonne sur gel de silice pour séparer le carbinol (R)-(+)-5-(phényl)hydroxyméthyl-1-méthyl- 1H-pyrazole, (R)-(+)-1, de l'ester (S)-(-)-5-(phényl) méthylcarbonyl-oxyméthyl-1-méthyl-1H-pyrazole, de formule (S)-(-)-4 dans laquelle R₁ représente un radical méthyle.

6. Procédé selon l'une des revendications 1 et 2, qui se caractérise en ce que l'on effectue l'hydrolyse de l'ester (S)-(-)-5-(phényl) alkylcarbonyl-oxyméthyl-1-méthyl-1H-pyrazole, de formule (S)-(-)-4 dans laquelle R₁ représente un radical méthyle ou éthyle, en milieu acide, pour obtenir le mélange racémique (±)-5-(phényl)hydroxyméthyl-1-méthyl-1H-pyrazole, de formule 1, qui à son tour peut être soumis à nouveau à un procédé de séparation, conformément aux revendications 1 et 2, en répétant le cycle autant de fois qu'il s'avère nécessaire.

7. Procédé selon l'une des revendications 1 et 2, qui se caractérise en ce que l'on effectue l'hydrolyse avec rétention de la configuration en milieu basique, de l'ester (S)-(-)-5-(phényl)alkylcarbonyloxyméthyl1-méthyl- 1H-pyrazole, de formule (S)-(-)-4, dans laquelle R₁ représente un radical méthyle ou éthyle, pour obtenir le (S)-(-)-5-(phényl)hydroxy-méthyl-1-méthyl-1H-pyrazole, de formule (S)-1.

## Claims

1. Process for preparing preponderantly the enantiomer (R)-(+)-5-(phenyl)hydroxymethyl-1-methyl-1H-pyrazole, (R)-(+)-1, by separation of the racemic mixture (±)-5-(phenyl)hydroxymethyl-1-methyl-1H-pyrazole, of formula 1, which is characterized by a sequential process which comprises the use of a lipase obtained from a microorganism in a transesterification reaction between the racemic mixture of formula 1 and an ester of formula 3 in which R₁ represents a methyl or ethyl radical and R₂ represents a vinyl or isopropenyl radical, as well as the hydrolysis of the ester formed, (S)-(-)-5-(phenyl)alkylcarbonyloxymethyl-1-methyl-1H-pyrazole, of formula (S)-(-)-4 in which R₁ represents a methyl or ethyl radical

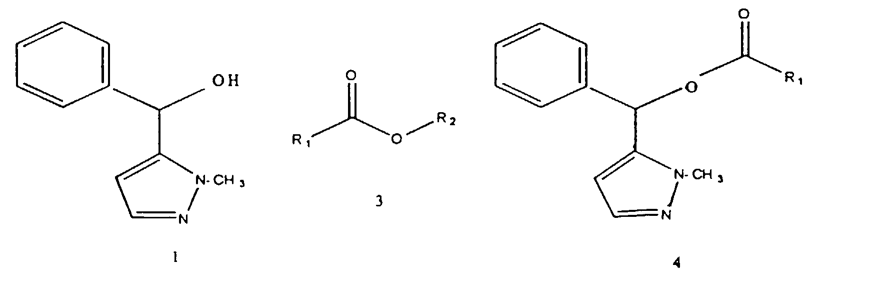

2. Process according to Claim 1, which is characterized by the use of a lipase obtained from a microorganism to act as catalyst in a transesterification reaction between an ester of formula 3, in which R₁ represents a methyl or ethyl radical and R₂ represents a vinyl or isopropenyl radical, and the enantiomer (S)-(-)-5-(phenyl)hydroxymethyl-1-methyl-1H-pyrazole, (S)-(-)-1, which gives rise to the formation of (S)-(-)-5-(phenyl)alkylcarbonyloxymethyl-1-methyl-1H-pyrazole, of formula (S)-(-)-4 in which R₁ represents a methyl or ethyl radical, and recovering the unreacted enantiomer (R)-(-)-5-(phenyl)hydroxymethyl-1-methyl-1H-pyrazole, (R)-(+)-1,

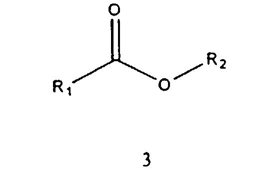

3. Process according to either of Claims 1 and 2, which is characterized by the use of vinyl acetate of formula 3, in which R₁ represents a methyl radical and R₂ represents a vinyl radical, as reactant and as solvent in the transesterification reaction.

4. Process according to one of Claims 1, 2 and 3, which is characterized by the use of a solvent of low polarity such as, for example, cyclohexane, to crystallize the enantiomer (R)-(+)-5-(phenyl)hydroxy-methyl-1-methyl-1H-pyrazole, (R)-(+)-1, from the solution which contains, in addition, the ester (S)-(-)-5-(phenyl)methylcarbonyloxymethyl-1-methyl-1H-pyrazole, of formula (S)-(-)-4 in which R₁ represents a methyl radical.

5. Process according to one of Claims 1, 2 and 3, which is characterized in that column chromatography on silica gel is performed to separate the carbinol (R)-(+)-5-(phenyl)hydroxymethyl-1-methyl-1H-pyrazole, (R)-(+)-1, from the ester (S)-(-)-5-(phenyl)methyl-carbonyloxymethyl-1-methyl-1H-pyrazole, of formula (S)-(-)-4 in which R₁ represents a methyl radical.

6. Process according to either of Claims 1 and 2, which is characterized in that the hydrolysis of the ester (S)-(-)-5-(phenyl)alkylcarbonyloxymethyl-1-methyl-1H-pyrazole, of formula (S)-(-)-4 in which R₁ represents a methyl or ethyl radical, is performed in an acid medium, to obtain the racemic mixture (±)-5-(phenyl)hydroxymethyl-1-methyl-1H-pyrazole, of formula 1, which, in its turn, can be subjected again to a process of separation in accordance with claims 1 and 2, repeating the cycle as many times as proves necessary.

7. Process according to either of Claims 1 and 2, which is characterized in that the hydrolysis of the ester (S)-(-)-5-(phenyl)alkylcarbonyloxymethyl-1-methyl-1H-pyrazole, of formula (S)-(-)-4, in which R₁ represents a methyl or ethyl radical, is performed with retention of the configuration in a basic medium, to obtain (S)-(-)-5-(phenyl)hydroxymethyl-1-methyl-1H-pyrazole, of formula (S)-1.

## Patentansprüche

1. Verfahren zur überwiegenden Herstellung des Enantiomeren (R)-(+)-5-(Phenyl)hydroxymethyl-1-methyl-1H-pyrazol, (R)-(+)-1, durch Trennung der racemischen Mischung (±)-5-(Phenyl)hydroxymethyl-1-methyl-1H-pyrazol der Formel 1, gekennzeichnet durch ein sequenzielles Verfahren, das die Verwendung einer aus einem Mikroorganismus stammenden Lipase in einer Umesterungsreaktion zwischen einer racemischen Mischung der Formel 1 und einem Ester der Formel 3, bei der R₁ einen Methyl- oder Ethylrest darstellt und R₂ einen Vinyl- oder Isopropenylrest darstellt, sowie die Hydrolyse des gebildeten Esters, (S)-(-)-5-(Phenyl)alkylcarbonyloxymethyl-1-methyl-1H-pyrazol der Formel (S)-(-)-4, in der R₁ einen Methyl- oder Ethylrest darstellt, umfaßt.

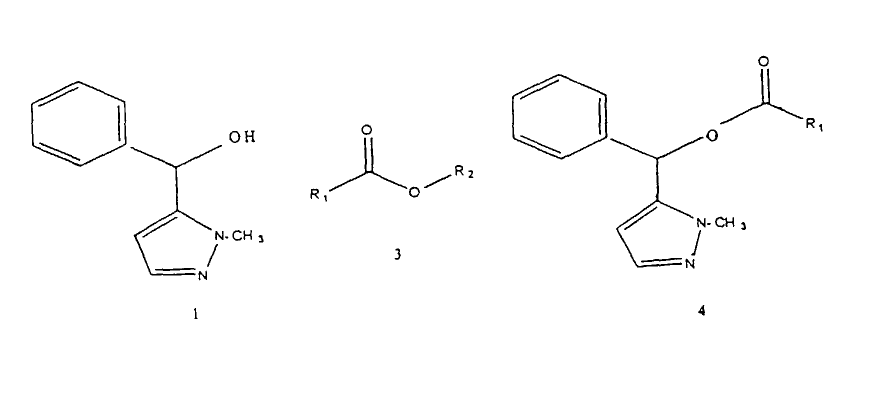

2. Verfahren nach Anspruch 1, charakterisiert durch die Verwendung einer aus einem Mikroorganismus stammenden Lipase, die als Katalysator in einer Umesterungsreaktion zwischen einem Ester der Formel 3, bei dem R₁ einen Methyl- oder Ethylrest darstellt und R₂ einen Vinyl- oder Isopropenylrest darstellt, und dem Enantiomeren (S)-(-)-5-(Phenyl)hydroxymethyl-1-methyl-1H-pyrazol, (S)-(-)-1 wirkt, was zur Bildung von (S)-(-)-5-(Phenyl)alkylcarbonyloxymethyl-1-methyl-1H-pyrazol der Formel (S)-(-)-4 führt, in der R₁ einen Methyl- oder Ethylrest darstellt, und durch Isolierung des Enantiomeren (R)-(-)-5-(Phenyl)hydroxymethyl-1-methyl-1H-pyrazol (R)-(+)-1 ohne Reaktion.

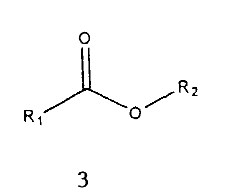

3. Verfahren nach einem der Ansprüche 1 und 2, charakterisiert durch Verwendung des Vinylacetats der Formel 3, in der R₁ einen Methylrest darstellt und R₂ einen Vinylrest darstellt, als Reagens und als Lösungsmittel in der Umesterungsreaktion.

4. Verfahren nach einem der Ansprüche 1, 2 und 3, charakterisiert durch Verwendung eines wenig polaren Lösungsmittels, wie beispielsweise Cyclohexan, zur Kristallisation des Enantiomeren (R)-(+)-5-(Phenyl)hydroxymethyl-1-methyl-1H-pyrazol, (R)-(+)-1, aus der Lösung, die darüber hinaus den Ester (S)-(-)-5-(Phenyl)methylcarbonyloxymethyl-1-methyl-1H-pyrazol der Formel (S)-(-)-4 enthält, in der R₁ einen Methylrest darstellt.

5. Verfahren nach einem der Ansprüche 1, 2 und 3, dadurch charakterisiert, daß man eine Chromatographie auf einer Kieselgelsäule zur Abtrennung des Carbinols (R)-(+)-5-(Phenyl)hydroxymethyl-1-methyl-1H-pyrazol, (R)-(+)-1, vom Ester (S)-(-)-5-(Phenyl)methylcarbonyloxymethyl-1-methyl-1H-pyrazol der Formel (S)-(-)-4 durchführt, in der R₁ einen Methylrest darstellt.

6. Verfahren nach einem der Ansprüche 1 und 2, dadurch charakterisiert, daß man die Hydrolyse des Esters (S)-(-)-5-(Phenyl)-alkylcarbonyloxymethyl-1-methyl-1H-pyrazol der Formel (S)-(-)-4, in der R₁ einen Methyl- oder Ethylrest darstellt, in saurem Medium durchführt, um die racemische Mischung (±)-5-(Phenyl)hydroxymethyl-1-methyl-lH-pyrazol der Formel 1 zu erhalten, die anschließend aufs neue einem Abtrennungsverfahren entsprechend den Ansprüchen 1 und 2 unterzogen werden kann, indem der Zyklus so oft wie notwendig wiederholt wird.

7. Verfahren nach einem der Ansprüche 1 und 2, dadurch charakterisiert, daß man die Hydrolyse des Esters (S)-(-)-5-(Phenyl)alkylcarbonyloxymethyl-1-methyl-1H-pyrazol der Formel (S)-(-)-4, in der R₁ einen Methyl- oder Ethylrest darstellt, unter Retention der Konfiguration in basischem Medium durchführt, um (S)-(-)-5-(Phenyl)hydroxymethyl-1-methyl-1H-pyrazol der Formel (S)-1 zu erhalten.
